# EUROPEAN PATENT APPLICATION

(11) **EP 4 831 281 A1**
(43) Date of publication of application: **28.10.2026**
(21) Application number: 24914554.1
(22) Date of filing: 03.01.2024
(51) Int. Cl.: C12N 5/0775, A61L 27/38, A61L 27/50, A61P 9/10

(54) **USE OF CELL SHEET FRAGMENTS IN TREATING LOWER LIMB ISCHEMIA**

(71) Applicant: Boe Technology Group Co., Ltd., Beijing 100015 (CN); BOE Regenerative Medicine Technology Co., Ltd., Beijing 100015 (CN); Beijing BOE Technology Development Co., Ltd., Daxing District Beijing 100176 (CN)
(72) Inventor: YANG, Xiaotong, Beijing 100176 (CN); MA, Jianlin, Beijing 100176 (CN); LIU, Shuai, Beijing 100176 (CN); LIU, Donghua, Beijing 100176 (CN); SHI, Lu, Beijing 100176 (CN); WANG, Juan, Beijing 100176 (CN); WANG, Jing, Beijing 100176 (CN); JIANG, Xiaotian, Beijing 100176 (CN)
(74) Representative: Durán-Corretjer, S.L.P.
(86) International application number: PCT/CN2024/070314
(87) International publication number: WO 2025/145321

(57) **Abstract**

The use of cell sheet fragments in the preparation of a drug used for treating lower limb ischemia. Specifically, the cell sheet fragments formed after injection administration of a cell sheet can prevent limb necrosis caused by lower limb ischemia, have good limb salvage effect, and also can effectively prevent muscle atrophy of affected limbs. In addition, the effect of limb salvage and the effect of preventing muscle atrophy of affected limbs of the cell sheet fragments are all equivalent to that of the cell sheet, and are remarkably superior to that of single-cell suspensions.

## Description

### Technical Field

This disclosure relates to the fields of biomedicine and cell biology, and in particular, to a use of cell sheet fragments in the treatment of lower limb ischemia.

### Background

Lower limb ischemia is a common peripheral arterial disease. It refers to a group of diseases characterized by stenosis or occlusion of the lower limb arteries and insufficient blood perfusion due to various etiologies, which further manifest as symptoms such as intermittent claudication, pain, ulceration or gangrene in the lower limbs. With the intensification of population aging, as well as lifestyles such as long-term smoking, type II diabetes mellitus, obesity, and sedentary, the risk of developing this disease has continued to increase in recent years. Lower limb ischemia, when it progresses to an advanced stage, carries a high risk of amputation and mortality. For patients who do not meet the indications for revascularization surgery, there are currently no effective treatment modalities. In recent years, mesenchymal stem cells have been confirmed to restore tissue function and repair ischemic tissue through immune regulation, promotion of angiogenesis, paracrine secretion of bioactive factors and other effects, and have been used to treat lower limb ischemia. Globally, an Indian company, Stempeutics' allogeneic bone marrow mesenchymal stem cell injection product REGENACIP^{®} has been launched in India for the treatment of lower limb ischemia. However, cell injection solutions have problems such as poor cellular resistance to acidic and ischemic microenvironment, short survival time, severe cell loss, insufficient accumulation and therapeutic action at lesion sites, and low cell utilization efficiency, resulting in unsatisfactory therapeutic effects. In recent years, there have also been studies on using biodegradable materials as scaffolds for stem cell transplantation. However, the interaction between the material and the cell, the degradation rate of the scaffold material, the tissue compatibility of the scaffold material and its degradation products, and so on all affect the final therapeutic effects. Therefore, mesenchymal stem cell preparations that can effectively treat lower limb ischemia still require further research and exploration.

### Summary

Cell sheet technology enables cells to connect with each other during culture through their own secreted extracellular matrix and form a two-dimensional sheet structure without external scaffolds. This effectively solves the problem of in situ cell retention, increases the number of surviving cells and cell survival time, and promotes the repair of lesion sites by secreting more and longer-lasting cytokines, thereby enhancing the therapeutic effects. Based on this, for those skilled in the art, cell sheets are usually administered via direct transplantation to ensure the uniformity of cell sheet size and structural integrity during administration, and to avoid structural damage. However, the inventors of the present disclosure were surprised to find in experimental studies that the cell sheet fragment formed after the cell sheet of the present disclosure is administered by injection can prevent limb necrosis caused by lower limb ischemia, achieve a good limb-salvage effect, and can also effectively prevent muscular atrophy of the affected limb. Moreover, the limb-salvage effect and the effect of preventing muscular atrophy of the affected limb and those of the cell sheet are comparable, and both are significantly better than those of single-cell suspension. It is evident that the cell sheet fragment formed after injection of the cell sheet of the present disclosure exhibit excellent therapeutic effects on lower limb ischemia and have broad application prospects.

To this end, in a first aspect of the present disclosure, use of a cell sheet fragment in the preparation of a medicament for the treatment of lower limb ischemia is provided.

In a second aspect of the present disclosure, the cell sheet fragment is provided, which is used for the treatment of the lower limb ischemia.

In a third aspect of the present disclosure, a method for treatment of the lower limb ischemia is provided, including administering an effective amount of the cell sheet fragment to a subject in need thereof.

In some embodiments, an administration route of the cell sheet fragment is injection administration.

In some embodiments, the injection administration is administered via intramuscular (such as into the adductor or gastrocnemius muscle) injection administration.

In some embodiments, when administering by injection, a dispersant for the cell sheet fragment is physiological saline.

In some embodiments, the cell sheet fragment is obtained by disrupting cell sheets.

In some embodiments, the cell sheet fragment has a diameter of about 0.1-3 mm, for example, about 0.1-0.2 mm, 0.2-0.3 mm, 0.3-0.4 mm, 0.4-0.5 mm, 0.5-0.6 mm, 0.6-0.7 mm, 0.7-0.8 mm, 0.8-0.9 mm, 0.9-1 mm, 1-1.1 mm, 1.1-1.2 mm, 1.2-1.3 mm, 1.3-1.4 mm, 1.4-1.5 mm, 1.5-1.6 mm, 1.6-1.7 mm, 1.7-1.8 mm, 1.8-1.9 mm, 1.9-2 mm, 2-2.1 mm, 2.1-2.2 mm, 2.2-2.3 mm, 2.3-2.4 mm, 2.4-2.5 mm, 2.5-2.6 mm, 2.6-2.7 mm, 2.7-2.8 mm, 2.8-2.9 mm or 2.9-3 mm. It should be noted that the diameter of the cell fragment refers to the longest line segment connecting two non-adjacent vertices within each cell fragment.

In some embodiments, the diameter of the cell sheet fragment is about 0.1-0.5 mm or about 0.4-3 mm.

In some embodiments, the cell sheet fragment is obtained by injecting a cell sheet via a syringe needle.

In some embodiments, the cell sheet fragment is obtained by the following method:
1) loading a cell sheet into a syringe needle;
2) after drawing physiological saline into a syringe barrel, mounting the syringe needle loaded with cell sheet onto the syringe barrel;
3) pushing the plunger of the syringe to inject the cell sheet to obtain the cell sheet fragment;
optionally, in step 1), the cell sheet is pre-cut (to accommodate different drug dosages).

In some embodiments, the needle has a gauge of 22G, 23G, 25G, or 27G. The needle gauge used in the present disclosure adopts international standards.

In some embodiments, the needle has a gauge size of 22G or 27G.

In some embodiments, the number of the cell sheet fragments is not less than 2, for example, not less than 3, not less than 4, not less than 5, not less than 6, not less than 7, not less than 8, not less than 9, not less than 10, not less than 12, not less than 14, not less than 16, not less than 18, not less than 20, not less than 22, not less than 24, not less than 26, not less than 28, not less than 30 or more.

In some embodiments, the cell is a mesenchymal stem cell.

In some embodiments, the cell is selected from the group consisting of an umbilical cord mesenchymal stem cell, a placental mesenchymal stem cell, an adipose mesenchymal stem cell, a bone marrow mesenchymal stem cell, an endometrial mesenchymal stem cell, and a dental pulp mesenchymal stem cell.

In some embodiments, the cell is an umbilical cord mesenchymal stem cells.

In some embodiments, the cell sheet includes multiple layers of cells, such as at least 2 layers, at least 3 layers, at least 4 layers, at least 5 layers, at least 6 layers, at least 7 layers, at least 8 layers, at least 9 layers, at least 10 layers, at least 12 layers, at least 14 layers, at least 16 layers, at least 18 layers, at least 20 layers, or more layers.

In some embodiments, the diameter of the cell sheet is about 3-30 mm, for example 3-4 mm, 4-5 mm, 5-6 mm, 6-7 mm, 7-8 mm, 8-9 mm, 9-10 mm, 10-12 mm, 12-14 mm, 14-16 mm, 16-18 mm, 18-20 mm, 20-22 mm, 22-24 mm, 24-26 mm, 26-28 mm, or 28-30 mm.

In some embodiments, the thickness of the cell sheet is about 50-1000 µm, such as 50-60 µm, 60-70 µm, 70-80 µm, 80-90 µm, 90-100 µm, 100-120 µm, 120-140 µm, 140-160 µm, 160-180 µm, 180-200 µm, 200-250 µm, 250-300 µm, 300-350µm, 350-400µm, 400-450µm, 450-500µm, 500-550µm, 550-600µm, 600-650µm, 650-700µm, 700-750µm, 750-800µm, 800-850µm, 850-900µm, 900-950µm, or 950-1000µm.

In some embodiments, the shape of cell sheet is circular or approximately circular.

In some embodiments, the cell sheet is capable of secreting one or more selected from interleukin-6 (IL-6), transforming growth factor-β (TGF-β), prostaglandin E2 (PGE2), hepatocyte growth factor (HGF), epidermal growth factor, fibroblast growth factor, platelet-derived growth factor, vascular endothelial growth factor (VEGF), insulin growth factor, stromal cell-derived growth factor-1, tryptophan metabolic enzyme (indoleamine 2,3-dioxygenase, IDO), and nitric oxide synthase (inducible nitric oxide synthase, iNOS).

In some embodiments, the cell sheet is capable of secreting at least interleukin-6 (IL-6), hepatocyte growth factor (HGF), and vascular endothelial growth factor (VEGF).

In some embodiments, the cell sheet includes extracellular matrix secreted by the cell.

In some embodiments, the extracellular matrix in the cell sheet includes one or more of fibronectin, integrin family (such as integrin-β1), and vitronectin.

In some embodiments, the extracellular matrix in the cell sheet includes at least fibronectin and integrin-β1.

In some embodiments, the volume of the physiological saline is 0.05-1 mL (e.g., 0.05-0.06 mL, 0.06-0.07 mL, 0.07-0.08 mL, 0.08-0.09 mL, 0.09-0.1 mL, 0.1-0.2 mL, 0.2-0.3 mL, 0.3-0.4 mL, 0.4-0.5 mL, 0.5-0.6 mL, 0.6-0.7 mL, 0.7-0.8 mL, 0.8-0.9 mL or 0.9-1 mL, preferably 0.1 mL).

In some embodiments, the cell sheet is obtained by the following method:
1) coating a surface of a temperature-sensitive culture dish with a layer of matrix;
2) adding a cell suspension to the temperature-sensitive culture dish for culture; and
3) lowering the temperature, so that the cell and the extracellular matrix secreted by the cell detach in sheet form, to obtain the cell sheet.

In some embodiments, the cell is a mesenchymal stem cell.

In some embodiments, the cell is selected from an umbilical cord mesenchymal stem cell, a placental mesenchymal stem cell, an adipose mesenchymal stem cell, a bone marrow mesenchymal stem cell, an endometrial mesenchymal stem cell, and a dental pulp mesenchymal stem cell.

In some embodiments, the cell is an umbilical cord mesenchymal stem cell.

In some embodiments, the coating matrix is selected from collagen, gelatin, fibronectin, fibrinogen, cohesin, vitronectin, laminin, polyornithine, polylysine, and ornithine-lysine copolymer.

In some embodiments, the coating is carried out in an incubator with saturated humidity, and 1-10% CO₂ (e.g., 1-2%, 2-3%, 3-4%, 4-5%, 5-6%, 6-7%, 7-8%, 8-9%, or 9-10% CO₂, preferably 5% CO₂) at 35-40°C (e.g., 35-36°C, 36-37°C, 37-38°C, 38-39°C, or 39-40°C, preferably 37°C).

In some embodiments, the coating time is 0.5-48 h, for example 0.5-1 h, 1-2 h, 2-3 h, 3-4 h, 4-5 h, 5-6 h, 6-7 h, 7-8 h, 8-9 h, 9-10 h, 10-15 h, 15-20 h, 20-25 h, 25-30 h, 30-35 h, 35-40 h, 40-45 h, or 45-48 h.

In some embodiments, the cell density in the temperature-sensitive culture dish is 1.8 × 10⁴-1.9 × 10⁶/cm² (e.g., 1.8×10⁴-2×10⁴/cm², 2×10⁴-3×10⁴/cm², 3×10⁴-4×10⁴/cm², 4×10⁴-5×10⁴/cm², 5×10⁴-6×10⁴/cm², 6×10⁴-7×10⁴/cm², 7×10⁴-8×10⁴/cm², 8×10⁴-9×10⁴/cm², 9×10⁴-1×10⁵/cm², 1×10⁵-2×10⁵/cm², 2×10⁵-3×10⁵/cm², 3×10⁵-4×10⁵/cm², 4×10⁵-5×10⁵/cm², 5×10⁵-6×10⁵/cm², 6×10⁵-7×10⁵/cm², 7×10⁵-8×10⁵/cm², 8×10⁵-9×10⁵/cm², 9×10⁵-1×10⁶/cm², 1×10⁶-1.5×10⁶/cm², or 1.5×10⁶-1.9×10⁶/cm², preferably 1×10⁶/cm²).

In some embodiments, step 2) is performed as follows: adding the cell suspension and a culture solution to the temperature-sensitive culture dish for culture.

In some embodiments, in step 2), the culture solution is a serum-free culture medium.

In some embodiments, the serum-free culture medium can be a basal culture medium plus nutrient additives, or a commercial serum-free culture medium.

In some embodiments, the basal culture medium may be 1640, DMEM, α-MEM, DMEM/F12, F12, and the like.

In some embodiments, the nutritional additives include a plurality of vitamin C, sodium selenate, hydrocortisone, insulin, transferrin, human serum albumin (plant expressed), progesterone, putrescine, biotin, sodium pyruvate, ethanolamine, L-carnitine, amino acids, vitamins, glutathione, linoleic acid, linolenic acid, and the like.

In some embodiments, the commercial serum-free culture medium includes, but is not limited to: CTSTMStemProTMMSC SFM Kit, MesenCultTM-ACF Medium, MesenCultTM-ACF Plus Medium, MesenCultTM-XF Medium, and the like.

In some embodiments, in step 2), a ratio of culture medium in the temperature-sensitive culture dish is 0.1-0.5 mL/cm² (e.g., 0.1-0.2 mL/cm², 0.2-0.3 mL/cm², 0.3-0.4 mL/cm² or 0.4-0.5 mL/cm², preferably 0.3 mL/cm²).

In some embodiments, in step 2), the culture is carried out in an incubator with saturated humidity, and 1-10% CO₂ (e.g., 1-2%, 2-3%, 3-4%, 4-5%, 5-6%, 6-7%, 7-8%, 8-9%, or 9-10% CO₂, preferably 5% CO₂) at 35-40°C (e.g., 35-36°C, 36-37°C, 37-38°C, 38-39°C, or 39-40°C, preferably 37°C).

In some embodiments, in step 2), the culture time is 2-48 h, for example 2-3 h, 3-4 h, 4-5 h, 5-6 h, 6-7 h, 7-8 h, 8-9, 9-10 h, 10-15 h, 15-20 h, 20-25 h, 25-30 h, 30-35 h, 35-40 h, 40-45 h, or 45-48 h.

In some embodiments, in step 3), the temperature is lowered to 4-32°C, for example, 4-5°C, 5-6°C, 6-7°C, 7-8°C, 8-9°C, 9-10°C, 10-12°C, 12-14°C, 14-16°C, 16-18°C, 18-20°C, 20-22°C, 22-24°C, 24-26°C, 26-28°C, 28-30°C, or 30-32°C.

In some embodiments, in step 3), no additional reagents or materials are added when the cells and extracellular matrix secreted by the cell detach in sheet form.

In a fourth aspect of the present disclosure, the present disclosure provides a use of cell sheet fragment in the preparation of a pharmaceutical composition for the treatment of lower limb ischemia.

In a fifth aspect of the present disclosure, the present disclosure provides a pharmaceutical composition including the cell sheet fragment for the treatment of lower limb ischemia.

In a sixth aspect of the present disclosure, the present disclosure provides a method for treatment of lower limb ischemia, including administering an effective amount of a pharmaceutical composition to a subject in need thereof, the pharmaceutical composition including the cell sheet fragment.

In some embodiments, the cell sheet fragment is as described in any of the above technical schemes.

In some embodiments, the cell is as described in any of the above technical schemes.

In some embodiments, the cell sheet is as described in any of the above technical schemes.

In some embodiments, the pharmaceutical composition further includes a pharmaceutically acceptable adjuvant, such as physiological saline.

In some embodiments, a dosage form of the pharmaceutical composition is an injection.

In some embodiments, the administration mode for the pharmaceutical composition is injection administration.

In some embodiments, the injection administration is intramuscular (such as into the adductor or gastrocnemius muscle) injection administration.

The present disclosure also provides a method for umbilical cord mesenchymal stem cells isolation and culture, which includes primary isolation and culture and passage culture.

In some embodiments, the primary isolation and culture includes the following steps: isolating Wharton's jelly from umbilical cord tissue; chopping the Wharton's jelly to obtain tissue blocks; plating the tissue blocks on a culture container for culture; adding an appropriate amount of complete culture medium to cover the tissue blocks and continuing culture; when cells attached to the culture container appear around the tissue blocks and the cells grow to 70-100% confluence, removing the tissue blocks and passaging the cells.

In some embodiments, the passage culture includes the following steps: separating the cells from the culture container and dispersing the cells evenly in the culture medium, and then seeding them into the culture container; adding an appropriate amount of culture medium, replacing it with an appropriate amount of fresh culture medium every 1 to 5 days according to the cell growth status, and repeating the passage operation when the cells grow to 70% to 100% confluence. Each time the cells are passaged, the passage number increases by 1. The umbilical cord mesenchymal stem cells grow adherently and exhibit a uniform, fibroblast-like morphology.

In the present disclosure, "treatment" generally refers to achieving the desired pharmacological and/or physiological effects, including: (a) suppressing the symptoms of a disease, i.e., preventing its progression; or (b) alleviating the symptoms of a disease, i.e., causing the disease or symptoms to deteriorate.

In the present disclosure, "subject" refers to vertebrates. In some embodiments, the vertebrates refer to mammals. The mammals include, but are not limited to, livestock (such as cattle), pets (such as cats, dogs, and horses), primates, mice, and rats. In some embodiments, the mammals refer to humans.

In the present disclosure, the term "effective amount" means an amount sufficient to achieve or at least partially achieve the desired effect. Determining such effective amount is entirely within the capabilities of those skilled in the art. For example, the effective amount for therapeutic purposes will depend on severity of disease being treated, an overall state of the patient's own immune system, a patient's general condition such as age, weight and sex, an administration mode of the drug, and other treatments being administered concurrently, etc. To further understand, for any specific individual, the specific administration regimen can be adjusted over time based on individual needs, the administration mode, or professional judgment of the personnel supervising the administration.

### Beneficial Effects

1. The cell sheet of the present disclosure can prevent limb necrosis caused by lower limb ischemia and achieve a good limb-salvage effect. At the same time, the cell sheet fragment can also prevent limb necrosis caused by lower limb ischemia and also has a good limb-salvage effect. In fact, the limb-salvage effect of the cell sheet fragment and that of the cell sheet are comparable, and both are significantly better than that of single-cell injection solution.
2. Both the cell sheet and the cell sheet fragment of the present disclosure can effectively prevent muscle atrophy in the affected limb, and the cell sheet fragment and the cell sheet are equally effective in preventing muscle atrophy in the affected limb, both being significantly superior to the single-cell injection solution. In summary, the cell sheet and the cell sheet fragment can significantly prevent muscle atrophy in affected limbs, which is beneficial for improving the function of ischemic limbs in the lower limbs, and the improvement effect increases with increasing dosage.
3. The cell sheet and the cell sheet fragment of the present disclosure can effectively improve the symptoms of lower limb ischemia, enhance blood perfusion in ischemic areas by promoting angiogenesis, prevent muscle atrophy in the affected limb, and help preserve the affected limb.

### Brief Description of Drawings

In FIG. 1, a shows a schematic diagram of an umbilical cord mesenchymal stem cell sheet; b shows a schematic diagram of the cell sheet being cut into 6 equal parts; c shows a schematic diagram of the cell sheet being loaded into a syringe before administration;
FIG. 2 shows a typical morphology of the cell sheet after injection via syringe needle (left: injection via 27G needle; right: injection via 22G needle);
FIG. 3 shows a blood perfusion status of ischemic limbs in mice improved by the umbilical cord mesenchymal stem cell sheet fragments detected by laser Doppler line scanning blood flow imager; and
FIG. 4 shows the gross appearance and gastrocnemius muscle morphology of the intact limb (left) and the affected limb (right) in the cell sheet fragment group and normal saline group at 28 days after administration.

### Detailed Description

The present disclosure will be further explained below with reference to specific embodiments, but in no way should it be construed as a limitation of the present disclosure.

The culture system of the present disclosure includes a serum-free culture medium and a coating matrix compatible with the culture medium, wherein:
1. Components of the serum-free culture medium can be a basal medium + nutrient additives, or a commercial serum-free culture medium, as detailed below:

### (1) The basal culture medium + the nutrient additives:

The basal culture medium can be 1640, DMEM, α-MEM, DMEM/F12, F12, and the like.

The nutritional additives include a plurality of vitamin C, sodium selenate, hydrocortisone, insulin, transferrin, human serum albumin (plant expressed), progesterone, putrescine, biotin, sodium pyruvate, ethanolamine, L-carnitine, amino acids, vitamins, glutathione, linoleic acid, linolenic acid, and the like.

### (2) The commercial serum-free culture medium:

Including but not limited to: CTSTMStemPro^{™}MSC SFM Kit, MesenCult^{™}-ACF Medium, MesenCult^{™}-ACF Plus Medium, MesenCult^{™}-XF Medium, and the like.

2. The coating matrix compatible with the culture medium:
Including but not limited to collagen, gelatin, fibronectin, fibrinogen, cohesin, vitronectin, laminin, polyornithine, polylysine, ornithine-lysine copolymer, and the like.

The culture system provided in the present disclosure is applicable to the culture of mesenchymal stem cells from various tissue sources, and preparation and detection of the cell sheet. The mesenchymal stem cells include but not limited to umbilical cord mesenchymal stem cells, placental mesenchymal stem cells, adipose mesenchymal stem cells, bone marrow mesenchymal stem cells, endometrial mesenchymal stem cells, and dental pulp mesenchymal stem cells.

The following will use the umbilical cord mesenchymal stem cells as an example only to further illustrate the present disclosure.

### Example 1: Isolation and Culture of the Umbilical Cord Mesenchymal Stem Cells

The umbilical cord mesenchymal stem cells were first subjected to primary isolation and culture and passage culture to achieve cell number expansion. The procedure was as follows:

### 1. Primary culture of the umbilical cord mesenchymal stem cells

The obtained neonatal umbilical cords (from Beijing Mingde Hospital, donated with informed consent from pregnant or postpartum women, and the informed consent form has been approved by the ethics committee of Mingde Hospital) were washed with a physiological solution with the same osmotic pressure as human body fluids, and arteries, veins, and adventitia were removed. Wharton's jelly was isolated, and the tissues were cut into small tissue blocks of 0.1-2 mm. The tissue blocks were evenly plated in a culture container coated with matrix, with a spacing of 2-30 mm between the tissue blocks. The culture container was placed in a cell culture incubator. After 2-7 days, appropriate amount of complete culture medium was added to cover the tissue blocks. The umbilical cord mesenchymal stem cells migrated out in 8-21 days. The migrated-out cells grew adherently and exhibited a uniform, fibroblast-like morphology. Once the migrated-out cells grew to 70-100% confluence, the tissue blocks were removed and the cells were performed passage operation.

### 2. Passage of umbilical cord mesenchymal stem cells

The cells were passaged when the cells grew to 70-100% confluence: the cells were separated from the culture container by methods including but not limited to digestion with trypsin and similar substances, cell scraping, etc. The cells were dispersed in the culture medium by methods such as stirring and vortexing (including but not limited to), and seeded into the culture container at a density of 500-100,000/cm². The appropriate amount of culture medium was added, and it was replaced with the appropriate amount of fresh culture medium every 1-5 days according to the cell growth status. When the cells grew to 70-100% confluence, the passage operation was repeated. The cultured umbilical cord mesenchymal stem cells grew adherently and exhibited a uniform, fibroblast-like morphology.

### Example 2: Assay and Identification of Umbilical Cord Mesenchymal Stem Cells

The obtained umbilical cord mesenchymal stem cells were assayed and identified using the following methods:

### 1. Assay of growth curves of umbilical cord mesenchymal stem cells

Methods for determining growth curves include but are not limited to, the MTT assay, the WST assay, DNA content detection, ATP detection, etc. Here, the WST assay would be used as an example for further explanation. The umbilical cord mesenchymal stem cells were dispersed and seeded into culture plates at a certain density according to reagent instructions. The medium was changed according to the normal culture conditions. At fixed times each day for a period of time, cell activity was assayed according to the instructions to obtain data on cell activity or number. Cell viability was assayed daily using WST reagent over a 7-day period. The method involved adding WST reagent to the plate with cells in culture according to the recommended ratio in the instructions, incubating for a fixed time in a cell culture incubator, and then assayed the absorbance value of the liquid in the plate wells at a wavelength of 450 nm using a microplate reader or UV spectrophotometer. The value was positively correlated with the number of cells. The results showed that cell activity in the wells increased with prolonged culture time, suggesting that the number of cells increased with prolonged culture time.

### 2. Identification of umbilical cord mesenchymal stem cells

Methods for identifying umbilical cord mesenchymal stem cells include, but are not limited to, flow cytometry for detecting cell surface marker proteins, trilineage differentiation of umbilical cord mesenchymal stem cells, and PCR for detecting expressed genes, etc. Here, flow cytometry and three-line differentiation were taken as examples for illustration.

### 2.1 Identification of surface markers of umbilical cord mesenchymal stem cells

After dispersing umbilical cord mesenchymal stem cells in a culture medium and centrifuging, the cell surface marker proteins were stained using serum or a physiological solution with a serum protein content of 1-20% that has the same osmotic pressure as human body fluids, according to the instructions of the purchased reagents. These cell surface marker proteins include, but are not limited to, CD73, CD90, CD105, CD34, CD11B, CD19, CD45, and HLA-DR. Among these, the phenotypes of CD73, CD90, and CD105 are positive, which should have proportions of not less than 95%, and the phenotypes of CD34, CD11B, CD19, CD45, and HLA-DR are negative, which should have proportions of not more than 2%. The results showed that the proportions of positive markers were all greater than 99% and the proportions of negative markers were all less than 0.1%.

### 2.2 Trilineage induced differentiation of umbilical cord mesenchymal stem cells

Umbilical cord mesenchymal stem cells have the ability to undergo induced to differentiate towards osteogenic, chondrogenic and adipogenic lineages. The specific operations for assaying osteogenic and adipogenic differentiation were as follows: cells were seeded into appropriate culture dishes according to the proportions specified in the instructions of the trilineage induced differentiation reagent. When the cells for osteogenic induction assay grew to 50-90% confluence and the cells for adipogenic induction assay grew to more than 90% confluence, osteogenic and adipogenic induction culture media were added respectively. During chondrogenic induction, a certain number of cells were centrifuged to the bottom of a centrifuge tube, and then chondrogenic induction medium was added. After the cells formed a pellet, the cell pellet was removed from the bottom of the tube to ensure complete contact with the induction medium. The cells were then detected after induction culture for more than 7 days. Osteogenic induction can be detected using a staining including but not limited to, Alizarin Red and anti-hOsteocalcin; adipogenic induction can be detected using a staining including but not limited to, Oil Red O and anti-mFABP4; and chondrogenic induction can be detected using a staining including but not limited to, Alcian Blue, Safranin O and anti-hAggrecan. The results showed that umbilical cord mesenchymal stem cells could be stained after undergoing adipogenic, osteogenic, and chondrogenic induction differentiation.

### Example 3: Preparation of Umbilical Cord Mesenchymal Stem Cell Sheet

The preparation procedure for umbilical cord mesenchymal stem cell membranes was as follows:

### 1. Temperature-sensitive smart culture dish coating

Before preparing the sheet using a serum-free culture system and mesenchymal stem cells, a matrix that facilitates the adhesion of mesenchymal stem cells should be pre-coated on the surface of the temperature-sensitive smart culture dish. The matrix include, but is not limited to, collagen, gelatin, fibronectin, fibrinogen, cohesin, vitronectin, laminin, polyornithine, polylysine, ornithine-lysine copolymer, etc. During coating, the coating matrix was diluted with physiological buffers such as physiological saline and PBS, and then added to a temperature-sensitive smart culture dish, and the culture dish was covered with its lid. Then, the temperature-sensitive smart culture dish was placed in a 37°C, saturated humidity, 5% CO₂ incubator for coating for 0.5-48 hours.

### 2. Preparation of umbilical cord mesenchymal stem cell sheet

After the coating was completed, the remaining coating matrix was removed from the smart culture dish, and mesenchymal stem cell suspension was added to the temperature-sensitive smart culture dish. In this example, a temperature-sensitive smart culture dish with a diameter of 35 mm was used, the amount of cells added was 1.8×10⁵-1.8×10⁷ (most preferably 1×10⁷), and the volume of culture medium added was 1-4 ml (most preferably 3 ml). Then, the temperature-sensitive smart culture dish was placed in a 37°C, saturated humidity, and 5% CO₂ incubator for 2-48 hours. After the culture was completed, the temperature-sensitive smart culture dish was moved to an environment of 4-32°C. The cells would detach from the bottom of the temperature-sensitive smart culture dish in a sheet manner without the need for any additional reagents or materials, thus becoming cell sheet. The liquid in the culture dish was collected to obtain the cell sheet supernatant. The cell sheet was off-white, had a dense structure with a smooth and flat surface. The dimensions were circular with a diameter of 3-30 mm and a thickness of 50-1000 µm. Tissue section observation revealed that the cell sheet had a multilayered cellular structure and contained extracellular matrix components secreted by the cells.

### Example 4: Functional assay of umbilical cord mesenchymal stem cell sheet

The functional assay on the formed cell sheet was performed as follows:

### 1. Enzyme-linked immunosorbent assay (ELISA) for the assay of factors secreted by the cell sheet

The supernatant from the cell sheet preparation process was assayed according to the instructions of the enzyme-linked immunosorbent assay kit to determine the content of various factors secreted by the cell sheet. These factors include, but are not limited to, interleukin-6 (IL-6), transforming growth factor-β (TGF-β), prostaglandin E2 (PGE2), hepatocyte growth factor (HGF), epidermal growth factor, fibroblast growth factor, platelet-derived growth factor, vascular endothelial growth factor (VEGF), insulin growth factor, stromal cell-derived growth factor-1, tryptophan metabolic enzyme (indoleamine 2,3-dioxygenase, IDO), and nitric oxide synthase (inducible nitric oxide synthase, iNOS). Here, we took interleukin-6 (IL-6), hepatocyte growth factor (HGF), and vascular endothelial growth factor (VEGF) as examples to assay the secretory factors of a certain umbilical cord mesenchymal stem cell sheet. The experiment was repeated in three samples (sample 1, sample 2, and sample 3). Furthermore, the secretory factors of human mesenchymal stem cell suspension and human mesenchymal stem cell sheet were compared using three samples. The results showed that human mesenchymal stem cell sheet had better factor secretion function compared with human mesenchymal stem cell suspension.

### 2. Observation of protein expression in cell sheet via tissue sectioning

The cell sheet obtained above was fixed with paraformaldehyde or formalin fixative, and then the cell sheet was made into tissue sections with a thickness of 4-10 µm by paraffin sectioning or frozen sectioning, and stained to observe the protein expression of the extracellular matrix in the cell sheet. The proteins include, but were not limited to, fibronectin, integrin family, vitronectin, etc. Usually, the cell nucleus was stained with fluorescent dyes such as DAPI or Hoechst 33258 at the same time to help with localization. In addition to immunofluorescence, staining and observation can also be performed using methods including but not limited to immunohistochemistry and the like. Here, we took fibronectin and integrin-β1 as examples for illustration. Both fibronectin and integrin-β1 were stained with antibody dyes labeled with fluorescein, and the cell nucleus was stained with DAPI. The results showed that the prepared cell sheet contained a large amount of fibronectin and integrin-β1.

### 3. Observation the surface structure of the cell sheet using scanning electron fiberscope

The cell sheet obtained above were fixed in 2.5% glutaraldehyde fixative, then dehydrated by gradient alcohol, and air-dried to obtain dried cell sheet for scanning electron microscopy analysis. The dried cell sheet was attached to the sample stage surface with conductive double-sided tape, and then the surface was sprayed with gold using vacuum magnetron sputtering to make the surface of the observed sample conductive. The sample stage was placed inside a scanning electron microscope (a Hitachi S-4800) for observation. The results showed that the cell sheet surface was relatively flat and smooth, and mesenchymal stem cells, intercellular protein junctions, and cell stacking were visible.

### Example 5: Activity assay of umbilical cord mesenchymal stem cell sheet and fragments thereof

### 1. Animal modeling:

(1) Mice of appropriate age (7 weeks in this experiment) (can be ICR/NODSCID/C57 male mice, ICR male mice in this experiment) were anesthetized by gas anesthesia with isoflurane or by intravenous injection with sodium pentobarbital or by intraperitoneal injection with hydrated aldehyde;
(2) After the mouse's hind limbs were fixed, shaved and disinfected, an incision of about 1 cm was made from the knee of the mouse's left leg to the inner thigh, and the subcutaneous tissues and muscle tissues were separated, to expose the blood vessels of the lower limbs;
(3) Modeling surgery method 1: From above the inguinal ligament to the bifurcation of the femoral artery, the two ends of the femoral artery were ligated with surgical sutures (nylon sutures, 7-0), and the collateral branches and the middle were cut off with a scalpel blade; Modeling surgery method 2: The femoral artery was separated with pointed forceps, and then the forceps were moved along the femoral artery to destroy the collateral branches and microvessels, and the proximal end of the femoral artery was ligated with surgical sutures (nylon sutures, 7-0);
(4) The incision was sutured with nylon sutures (4-0 sutures were used in this experiment);
(5) A laser Doppler line scanning blood flow imager (the instrument model in this experiment was: Moor LDLS) was used to detect blood flow signals and the postoperative blood flow ratio of the affected side/contralateral side was calculated;
(6) The animal was taken to the feeding area and allowed to wake up from anesthesia. Painkiller meloxicam was injected daily for three days after the surgery.

### 2. Administration of cell sheet:

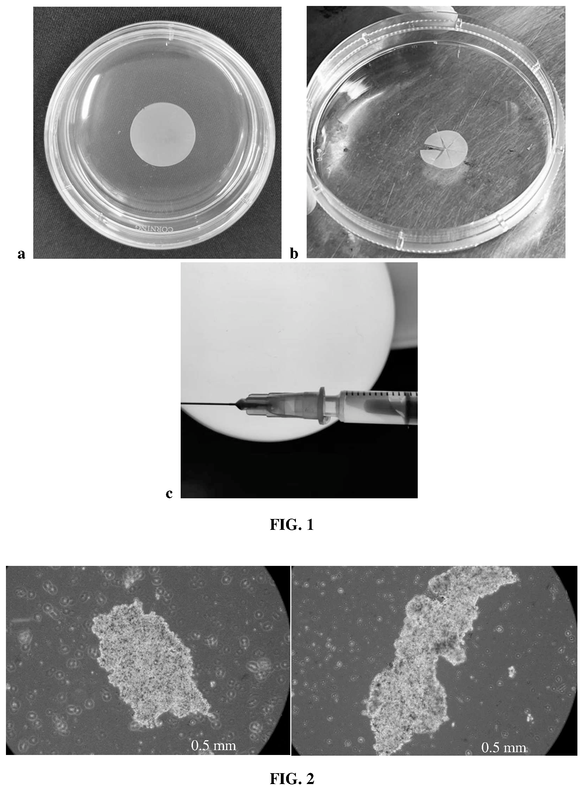

A single dose was administered within 0-72 hours after animal modeling surgery. Specific steps are as follows:
(1) After modeling, mice were anesthetized by gas anesthesia with isoflurane or by intravenous injection with sodium pentobarbital or by intraperitoneal injection with hydrated aldehyde, which was administered after local disinfection with iodine;
(2) The prepared cell sheet was cut into the required size according to the dosage. Taking mouse administration as an example, the cell sheet with a diameter of 1.8 cm was cut into 6 equal parts for administration;
(3) The administration method can be direct transplantation or injection: the direct transplantation method was as follows: after animal modeling step (3), before suturing the incision, the cell sheet was laid flat near the femoral artery, waited for 2 minutes, and then the incision was sutured to complete the administration; the injection method was as follows: the syringe needle (any gauge size of needle can be used; for example, a 27G needle can be used for mouse administration) was removed, the cut cell sheet was loaded into the syringe needle (see FIG. 1), 0.1 mL of physiological saline (0.05-1 mL is acceptable) was drew into the syringe barrel, and then the syringe needle loading with the cell sheet was gently mounted to the syringe barrel. The needle was inserted into the thigh muscle of the affected limb (which could be the adductor or gastrocnemius muscle; in this experiment, the adductor muscle was used). The syringe was pushed to inject the cell sheet into the mouse muscle through the needle. The needle was then withdrawn, and the administration was completed. (The size of the sheet fragments after being injected into the adductor muscle varies depending on the size of the syringe needle. For example, with a 27-gauge needle, the size of the sheet fragments after injection was approximately 0.1-0.5 mm; with a 22-gauge needle, the size of the sheet fragments after injection was approximately 0.4-3 mm. See FIG. 2.)

### 3. Effectiveness assay:

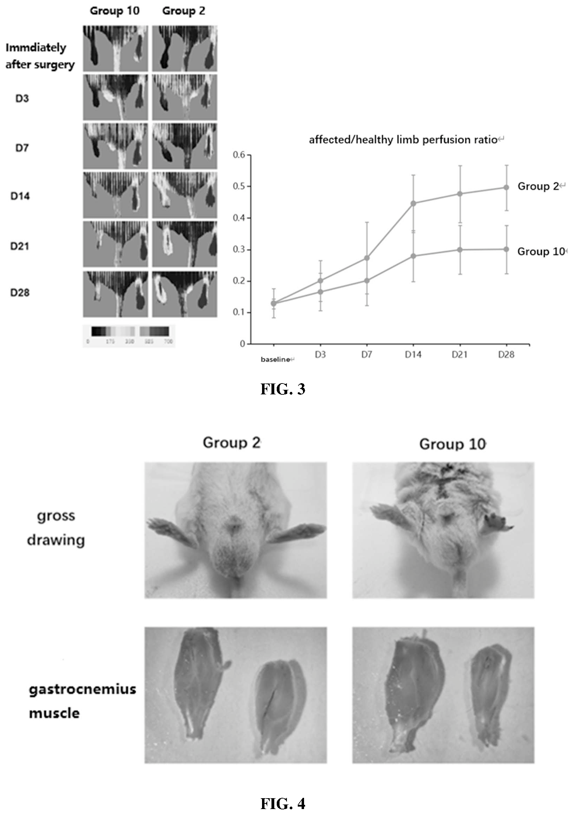

Multiple experimental groups with varying dosages were set up, including a low-dose group (equivalent to 1*10^6 cells) and a high-dose group (equivalent to 2*10^6 cells): (Group 1: low-dose cell sheet, Group 2: low-dose cell sheet fragment, Group 3: low-dose single-cell injection, Group 4: low-dose cell sheet supernatant; Group 5: high-dose cell sheet, Group 6: high-dose cell sheet fragment, Group 7: high-dose single-cell injection, Group 8: high-dose cell sheet supernatant). The improvement effect of each experimental group on lower limb ischemic disease was assayed. Animals that were modeled but not administrated (Group 9) or injected with an equal volume of physiological saline after modeling (Group 10) were used as experimental control groups to verify the effectiveness of umbilical cord mesenchymal stem cell sheet and fragment thereof in treating lower limb ischemia.
(1) Improvement of lower limb blood perfusion: Blood flow signals were detected by laser Doppler line scanning blood flow imager on days 3, 7, 14, 21, and 28 after administration. The postoperative blood flow ratio of the affected side/contralateral side was calculated to evaluate the improvement of lower limb blood perfusion. The comparison showed that the blood flow of the animal in the cell sheet fragment group (group 2) was significantly improved from day 14. The improvement was about 16.4% on day 14 compared with the control group (group 10), about 17.5% on day 21, and about 19.3% on day 28 (see FIG. 3).
(2) Evaluation of limb preservation effect: The limb damage of mice in the control group and the experimental group was observed on days 7, 14, 21 and 28 after administration. The proportions of mice in each group with normal or only toe discoloration, toe necrosis and foot ring death on the modeling side were calculated. The proportions of the three conditions in each group were statistically analyzed, and the proportions of the three conditions in group 1 to group 10 were as follows, respectively: Group 1: 80%, 12%, 8%; Group 2: 83%, 8%, 9%; Group 3: 58%, 28%, 14%; Group 4: 60%, 24%, 16%; Group 5: 87%, 8%, 5%; Group 6: 89%, 5%, 6%; Group 7: 68%, 18%, 14%; Group 8: 68%, 22%, 10%; Group 9: 50%, 25%, 25%; and Group 10: 45%, 23%, 32%. This shows that the cell sheet can prevent limb necrosis caused by lower limb ischemia and achieve a good limb-salvage effect. At the same time, the cell sheet fragment can also prevent limb necrosis caused by lower limb ischemia and achieve a good limb-savage effect. In fact, the limb-salvage effect of cell sheet fragment and that of the cell sheet are comparable, and both are significantly better than that of the single-cell injection group.
(3) Prevention of muscle atrophy in the affected limb: On day 28 after administration, the gastrocnemius muscles of the affected and healthy limbs of the mice were dissected (see FIG. 4). The weight of the gastrocnemius muscles on both sides was weighed and the weight ratio of the gastrocnemius muscles of the affected limb to the healthy limb was calculated. The weight ratios of the gastrocnemius muscles of the affected limb to the healthy limb in each group were 82.2%, 80.4%, 53.1%, 57.8%, 85.2%, 84.8%, 68.3%, 67.8%, 51.4%, and 50.2%, respectively. This shows that both the cell sheet and the cell sheet fragment can effectively prevent muscle atrophy in the affected limb, and the cell sheet fragment and the cell sheet are equally effective in preventing muscle atrophy in the affected limb, both being significantly better than the single-cell injection group. In summary, the cell sheet and the cell sheet fragment can significantly prevent muscle atrophy in affected limbs, which is beneficial for improving the function of ischemic limbs in the lower limbs, and the improvement effect increases with increasing dosage.

While specific embodiments of the present disclosure have been described above, those skilled in the art should understand that these are merely illustrative embodiments, and the scope of protection of the present disclosure is defined by the appended claims. Those skilled in the art can make various changes or modifications to these embodiments without departing from the principles and essence of the present disclosure, but all such changes and modifications fall within the protection scope of the present disclosure.

## Claims

1. Use of a cell sheet fragment in the preparation of a medicament for the treatment of lower limb ischemia.

2. The use according to claim 1, wherein the cell sheet fragment is obtained by disrupting a cell sheet.

3. The use according to any one of claims 1 to 2, wherein the diameter of the cell sheet fragment is about 0.1-3 mm, preferably about 0.1-0.5 mm or about 0.4-3 mm.

4. The use according to any one of claims 1 to 3, wherein the cell sheet fragment is obtained by injecting a cell sheet via a syringe needle.

5. The use according to any one of claims 1 to 4, wherein the cell sheet fragment is obtained by the following method:
1) loading a cell sheet into a syringe needle;
2) after drawing physiological saline into a syringe barrel, mounting the syringe needle loaded with the cell sheet onto the syringe barrel; and
3) pushing the plunger of the syringe to inject the cell sheet to obtain the cell sheet fragment; optionally, in step 1), the cell sheet is pre-cut.

6. The use according to any one of claims 4 to 5, wherein the needle has a gauge of 22G, 23G, 25G or 27G, preferably 22G or 27G.

7. The use according to any one of claims 1 to 6, wherein the number of the cell sheet fragments is not less than two.

8. The use according to any one of claims 1 to 7, wherein the cell is a mesenchymal stem cell, preferably selected from an umbilical cord mesenchymal stem cell, a placental mesenchymal stem cell, an adipose mesenchymal stem cell, a bone marrow mesenchymal stem cell, an endometrial mesenchymal stem cell, and a dental pulp mesenchymal stem cell, more preferably an umbilical cord mesenchymal stem cell.

9. The use according to any one of claims 1 to 8, wherein the cell sheet further has one or more technical features selected from (i)-(vi):
(i) the cell sheet comprises multiple layers of cells;
(ii) the diameter of the cell sheet is about 3-30 mm;
(iii) the thickness of the cell sheet is about 50-1000 µm;
(iv) the shape of the cell sheet is circular or approximately circular;
(v) the cell sheet is capable of secreting one or more selected from interleukin-6 (IL-6), transforming growth factor-β (TGF-β), prostaglandin E2 (PGE2), hepatocyte growth factor (HGF), epidermal growth factor, fibroblast growth factor, platelet-derived growth factor, vascular endothelial growth factor (VEGF), insulin growth factor, stromal cell-derived growth factor-1, tryptophan metabolic enzyme (indoleamine 2,3-dioxygenase, IDO), and nitric oxide synthase (inducible nitric oxide synthase, iNOS);
preferably, the cell sheet is capable of secreting at least interleukin-6 (IL-6), hepatocyte growth factor (HGF), and vascular endothelial growth factor (VEGF); and
(vi) the cell sheet comprises extracellular matrix secreted by the cell;
preferably, the extracellular matrix in the cell sheet comprises one or more of fibronectin, integrin family (such as integrin-β1), and vitronectin;
more preferably, the extracellular matrix in the cell sheet comprises at least fibronectin and integrin-β1.

10. The use according to any one of claims 1 to 9, wherein the cell sheet is obtained by the following method:
1) coating a surface of a temperature-sensitive culture dish with a layer of matrix;
2) adding a cell suspension to the temperature-sensitive culture dish for culture; and
3) lowering the temperature, so that the cell and the extracellular matrix secreted by the cell detach in sheet form, to obtain the cell sheet.

11. The use according to claim 10, wherein the method for preparing the cell sheet further comprises one or more technical features selected from (i)-(x):
(i) the cell is a mesenchymal stem cell, preferably selected from an umbilical cord mesenchymal stem cell, a placental mesenchymal stem cell, an adipose mesenchymal stem cell, a bone marrow mesenchymal stem cell, an endometrial mesenchymal stem cell, and a dental pulp mesenchymal stem cell, more preferably an umbilical cord mesenchymal stem cell;
(ii) the coating matrix is selected from the group consisting of collagen, gelatin, fibronectin, fibrinogen, cohesin, vitronectin, laminin, polyornithine, polylysine, and ornithine-lysine copolymer;
(iii) the coating is carried out in an incubator with saturated humidity and 1-10% CO₂ (preferably 5% CO₂) at 35-40°C (preferably 37°C);
(iv) the coating time is 0.5-48 h;
(v) the cell density in the temperature-sensitive culture dish is 1.8×10⁴-1.9×10⁶/cm² (preferably 1×10⁶/cm²);
(vi) step 2) is performed as follows:
adding the cell suspension and a culture solution to the temperature-sensitive culture dish for culture;
preferably, the culture solution is a serum-free culture medium;
preferably, the ratio of the culture solution in the temperature-sensitive culture dish is 0.1-0.5 mL/cm² (preferably 0.3 mL/cm²);
(vii) the culture is carried out in an incubator with saturated humidity and 1-10% CO₂ (preferably 5% CO₂) at 35-40°C (preferably 37°C);
(viii) the culturing time is 2-48 h;
(ix) in step 3), the temperature is lowered to 4-32°C; and
(x) no additional reagents or materials are added when the cell and the extracellular matrix secreted by the cell detach in sheet form.

12. Use of a cell sheet fragment in the preparation of a pharmaceutical composition for the treatment of lower limb ischemia.

13. The use according to claim 12, wherein the cell sheet fragment is as defined in any one of claims 1 to 7.

14. The use according to any one of claims 12 to 13, wherein the cell is as defined in claim 8.

15. The use according to any one of claims 12 to 14, wherein the cell sheet is as defined in any one of claims 9 to 11.

16. The use according to any one of claims 12 to 15, wherein the pharmaceutical composition further comprises a pharmaceutically acceptable adjuvant, such as physiological saline.

17. The use according to any one of claims 12 to 16, wherein the dosage form of the pharmaceutical composition is an injection.
